# EUROPEAN PATENT SPECIFICATION

(11) **EP 4 427 201 B1**
(45) Date of publication and mention of the grant of the patent: **18.11.2026**
(21) Application number: 22888692.5
(22) Date of filing: 04.11.2022
(51) Int. Cl.: G06V 10/44, G06V 10/778, G06V 10/98, G16H 30/40, G16H 50/70, G06N 20/00, G16H 50/20, A61F 9/008

(54) **HYBRID CLASSIFIER TRAINING FOR FEATURE ANNOTATION**
HYBRIDES KLASSIFIKATORTRAINING FÜR MERKMALSANNOTATION
APPRENTISSAGE DE CLASSIFICATEUR HYBRIDE POUR ANNOTATION DE CARACTÉRISTIQUES

(30) Priority: 05.11.2021 CA 3137612
(43) Date of publication of application: 11.09.2024
(73) Proprietor: Pulsemedica Corp., Edmonton, AB T5J 3E8 (CA)
(72) Inventor: KATCHINSKIY, Nir, Edmonton, Alberta T6J 2V2 (CA); CEROICI, Christopher, Edmonton, Alberta T5K 1K2 (CA)
(74) Representative: Cobbold, Alistair John
(86) International application number: PCT/CA2022/051638
(87) International publication number: WO 2023/077238

(56) References cited:
- WO-A1-2022/133590
- US-A1- 2017 132 826
- US-A1- 2020 250 436
- MASAHIRO MITSUHARA ET AL: "Embedding Human Knowledge into Deep Neural Network via Attention Map", ARXIV.ORG, CORNELL UNIVERSITY LIBRARY, 201 OLIN LIBRARY CORNELL UNIVERSITY ITHACA, NY 14853, 9 May 2019 (2019-05-09), XP081557804
- FUKUI HIROSHI ET AL: "Attention Branch Network: Learning of Attention Mechanism for Visual Explanation", ARXIV.ORG, 1 June 2019 (2019-06-01), pages 10705 - 10714, XP055866213, DOI: 10.1109/CVPR.2019.01096
- SELVARAJU RAMPRASAATH R ET AL: "Grad-CAM: Visual Explanations from Deep Networks via Gradient-based Localization", ARXIV.ORG, 3 December 2019 (2019-12-03), pages 1 - 23, XP093311164, Retrieved from the Internet <URL:https://arxiv.org/pdf/1610.02391> DOI: 10.48550/arxiv.1610.02391
- SATTARZADEH SAM ET AL: "Integrated Grad-Cam: Sensitivity-Aware Visual Explanation of Deep Convolutional Networks Via Integrated Gradient-Based Scoring", ICASSP 2021 - 2021 IEEE INTERNATIONAL CONFERENCE ON ACOUSTICS, SPEECH AND SIGNAL PROCESSING (ICASSP), IEEE, 6 June 2021 (2021-06-06), pages 1775 - 1779, XP033954155, DOI: 10.1109/ICASSP39728.2021.9415064

## Description

### TECHNICAL FIELD

The current disclosure relates to the automatic annotation of features present in images and in particular to training of models for performing the annotation.

### BACKGROUND

Medical images are often used to identify potential diseases or conditions. The images can be processed by a professional, or by a trained machine learning model. For example, image segmentation models take an image as input and output a line vector or image mask outlining a particular feature that the model was trained to identify. The particular features that the model is trained to identify can vary. For example, with medical images, the features can be associated with a disease or condition. While such image segmentation models can provide relatively accurate segmentation or extraction of the disease features, the training of the models require relatively large training data sets of input images that have had the particular features annotated. The annotation of features of the training images is often performed manually

Hand annotation of features in images to create training data sets can be impractical due to the large number of images necessary and/or the difficulty in annotation numerous small features. Without annotated features, the segmentation model may not be trained to extract features in unknown images.

While a segmentation model can be trained to extract features in images, a classification model can be trained to classify unknown images into one or more classifications. Classification models can be trained using a training set of images that have been labelled with the correct classification.

While classifying models and segmentation models can be useful, it is desirable to have an additional, alternative, and/or improved technique of training the models.

"Embedding Human Knowledge into Deep Neural Network via Attention Map" by Masahiro Mitsuhara et al. describes a method for embedding human knowledge into deep neural networks via attention mechanism of an attention branch network (ABN). A fine-tuning method that utilizes a single-channel attention map which is manually edited by a human expert is described, which can train a network so that the output attention map corresponds to the edited ones. The fine-tuned network can output an attention map that takes into account human knowledge.

### SUMMARY

The invention is defined by the appended claims.

### BRIEF DESCRIPTION OF THE DRAWINGS

Further features and advantages of the present disclosure will become apparent from the following detailed description, taken in combination with the appended drawings, in which:
FIG. 1 depicts training and using a machine learning classification model;
FIG. 2 depicts generating additional training images;
FIG. 3 depicts automatic disease feature annotation functionality;
FIG. 4 depicts a method of automatically annotating disease features in medical images;
FIG. 5 depicts a process for the hybrid training of the classification model used for feature extraction;
FIG. 6 depicts a process for retaining the model;
FIGs. 7A and 7B depict example medical images and feature maps;
FIGs. 8A and 8B depict example medical images and feature maps;
FIG. 9 depicts a method of training a model for automatically annotating images;
FIG. 10 depicts a further method of training a model for automatically annotating images; and
FIG. 11 depicts a system using the hybrid training of annotation models.

### DETAILED DESCRIPTION

Generating sets of training images for use in training segmentation models to automatically annotate features in images can be difficult and/or time consuming. Previously, individual images had to be manually annotated in order to identify the features within the images that are to be identified by the segmentation model. An automatic annotation system is described further below that can automatically extract and annotate features in images. The automatic annotation system can be used to generate large training sets required for training a segmentation model without having to manually annotate a large set of images. The following describes the annotation model and model training with particular reference to medical images of the eye; however, the same techniques can be used for the training of models for the automatic extraction of features from different types of images. The automatic feature extraction allows features, which can include features indicative of a particular disease, to be extracted from the images. As described further below, rather than using a trained segmentation model to extract the features, the process uses a trained classification model to identify the locations within the input images that cause the image to be classified as healthy vs diseased. Training the classification model only requires an identification of whether or not the image is indicative of a particular disease, which can be considerably less work than having to annotate individual features indicative of the disease within the images. The trained classification model used to annotate individual features may incorrectly identify features, either missing features or identifying areas that are not in fact features. In order to improve training of the classification model, a small subset of images may be manually annotated in order correct for any misidentification. The manually annotated subset of images may then be used to retrain the classifier.

The identified features can be further processed for example to automatically annotate individual features, which can in turn be used for various applications. For example, the annotated features identified by the trained annotation model can be used in diagnosis the disease, planning a treatment of the disease, and/or possibly treating the disease. I

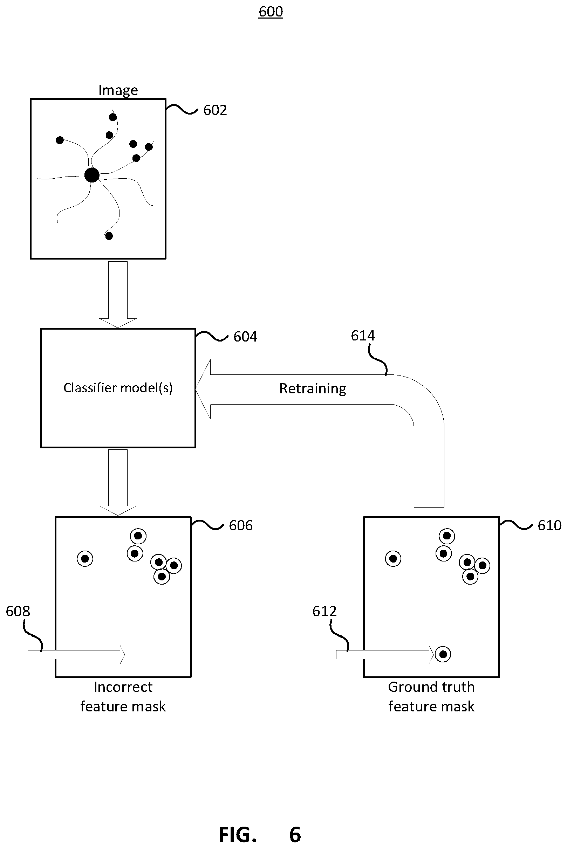

In cases where an abundance of input images are available, or can be prepared, with appropriate labels, such as "Healthy image", "Disease A image", "Disease B image", it is possible to use these labels to train a classification network that can then be used to provide the annotation/feature extraction output. Illustrative images associated with a patient with diabetic retinopathy and glaucoma is depicted in FIGs 6A - 7B. In addition to using the relatively large set of labelled images to train the classification model, a hybrid training process can also combine a relatively small set of annotated features or feature maps to train the classification model. As an example, the classification model can be trained using a large set of labelled images and then used to generate feature maps of the images which can be, for example, features associated with a particular disease or condition that cause the classification model to output the particular classification. A small set of the images and feature maps can be manually reviewed and corrected for any misidentified features. The corrected feature maps can then be used along with labelled images in training the model. The trained model can be deployed or stored to one or more computing devices that will implement and use the trained model. Similarly, once the trained model is deployed any corrections made by a user to the automatically generated feature maps can be used to retrain the classification model. The retrained model can be again deployed or stored to one or more computing systems.

The process for training, and re-training, annotation or classification models for use in identifying image features indicative of a disease condition is easier as it does not require the large training set of manually annotated features. The training of the automatic annotation model can be improved with a relatively small set of corrected feature maps. The trained annotation model can then applied to new images in order to identify locations of the features within the new images. Although the annotation model is described with particular reference to identifying disease features within images of the eye, the same process can be applied to identify features that are indicative of a particular classification, whether it is a disease or some other classification. Since the images used in training the annotation or classification models only need to be classified as either being indicative of the disease or not, the automatic annotation of features can identify possible features or biomarkers present in the images that were not previously known to be associated with the disease. That is, the disease or condition of a patient may be determined in other non-image based ways and then captured patient images labelled with the disease/condition. The trained classifier could identify possible disease indications present in the images.

The first step in training the automatic feature extraction is to train a classification model for one or more of the classification labels. The classification model can have any structure, but since a very high accuracy is desirable from the classification model, models can be chosen based on the best performing image classification models such as xception, resnext, or mnastnet. As an example, a model in accordance with the current disclosure that provides retina classification can be xception with additional layers added for image downscaling. The retina classification model was trained to 99.9% accuracy from 3,000 images with 2 class labels of "Healthy" and "Diabetic Retinopathy". In order to increase the training data available, training data augmentation can be used, which adjusts or modifies training images for example by rotating, stretching, mirroring, or adjusting other characteristics of the images to generate additional images. Data augmentation can help avoid or reduce overfitting the classification model to the available training images. The model being trained can be used to generate feature maps of the image features that lead to a particular classification of the image. A subset of the feature maps can be manually reviewed and any misidentified features corrected and the corrected feature map used in the further training of the model.

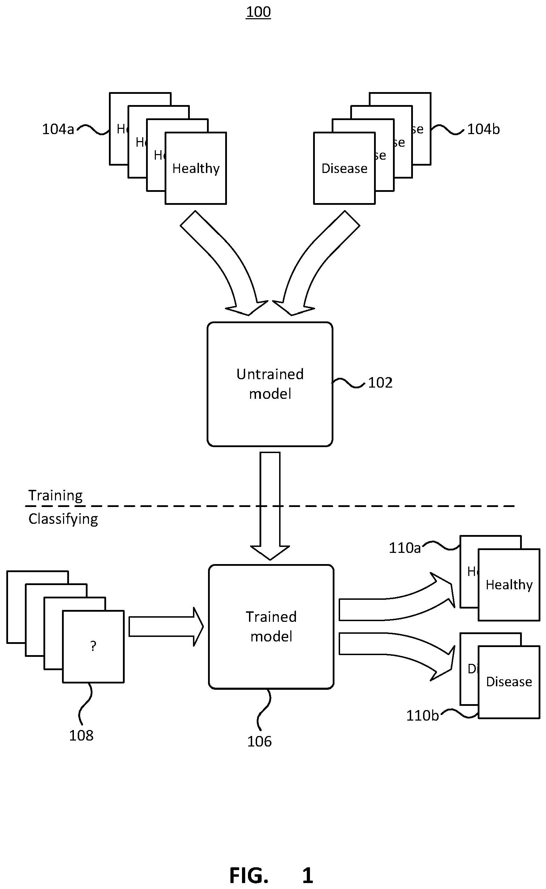

FIG. 1 depicts a classification model. As depicted, an untrained classification model 102 can be trained with a plurality of images that have been labeled as either healthy 104a, or disease 104b. Similar to the training of the segmentation model in which individual features present in the image are manually annotated or outlined, the training images are labelled as either representing a healthy condition, or a disease condition. However, given that images are labelled as being either healthy or having a particular disease or condition present, generating a training dataset can be significantly easier since the individual features do not need to be identified. Once the model 102 has been sufficiently trained, the trained model 106 can be used to classify unknown images 108. The trained model 106 can classify the unknown images as either being healthy 110a or representative of a particular disease 110b. The trained model 106 can be trained to classifying one or more diseases or conditions.

After training the classification model, the trained model can be applied to unknown images in order to classify them as healthy or indicative of a disease such as diabetic retinopathy. In addition to classifying the image as healthy or diseased, the model can generate a feature map highlighting those features associated with the disease classification. The feature map that led to the particular disease classification can be used as a feature annotation of the image.

The trained classification model can generate the feature map using various techniques. For example, saliency is a technique which calculates the gradients of the input image for the classification model. The gradient indicates the change in the output for changes to the input. The saliency technique mathematically determines the changes in the model output based on input changes by determining the input gradient, or image gradient, of the classification model. The input gradient can highlight those areas, or features, of the image that were most important in generating the classification. The input gradient can be defined as: ${γ}_{ij} = \frac{\partial {p}_{k}}{\partial {x}_{ij}}$

Where:
- *γᵢⱼ* is the image gradient for an image *x* of pixels *xᵢⱼ*
- *pₖ* is a model output prediction for the class *k*

The trained classification model can be trained to output a prediction that the input image is associated with one or more particular classes the model has been trained to classify. The gradient can be calculated mathematically and be used directly for features extraction to identify the locations in the input image that have the largest impact on the classification. The gradient-based approach for feature extraction can be used to quantify the effects that each input pixel, or groups of pixels, has on a particular output. The amount that a change in an input pixel will change the output of interest can be calculated. For some input image *x* consisting of pixels *xᵢⱼ* it is possible to evaluate the model to obtain predictions *p* giving the probability that the particular pixel is associated with one of the trained classes. A feature map of those features that are highly indicative of a particular class can then be generated.

A variation of this gradient approach is called "integrated gradient" in which there is provided an approximation of an integration of: ${γ}_{ij} = {\int }_{a = 0}^{1} \frac{\partial {p}_{k}}{\partial \left({x}_{ij}\times a\right)} \frac{\partial \left({x}_{ij}\times a\right)}{\partial a} da$

In the above, *a* is factor used to scale the input *x.* This approach integrates the input gradients across evaluations of an input *x* scaled by some factor *a* swept from 0 to 1. This can be approximated with: ${γ}_{ij} ≅ {\sum }_{n = 1}^{m} \frac{\partial {p}_{k}}{\partial \left({x}_{ij}\times \left(\frac{n}{m}\right)\right)} \left({x}_{ij}\times \frac{n}{m}-{x}_{ij}\times \frac{n - 1}{m}\right)$

An advantage here is that the integrated gradients can give a more complete accounting of gradients contributing to the output probabilities. This also avoids the saturation issue, where "saturated" nodes can have a large contribution to an output but zero gradient.

Once the classification model has been trained, it can be used to not only classify unknown images but also generate a feature map highlighting the important features for the classification.

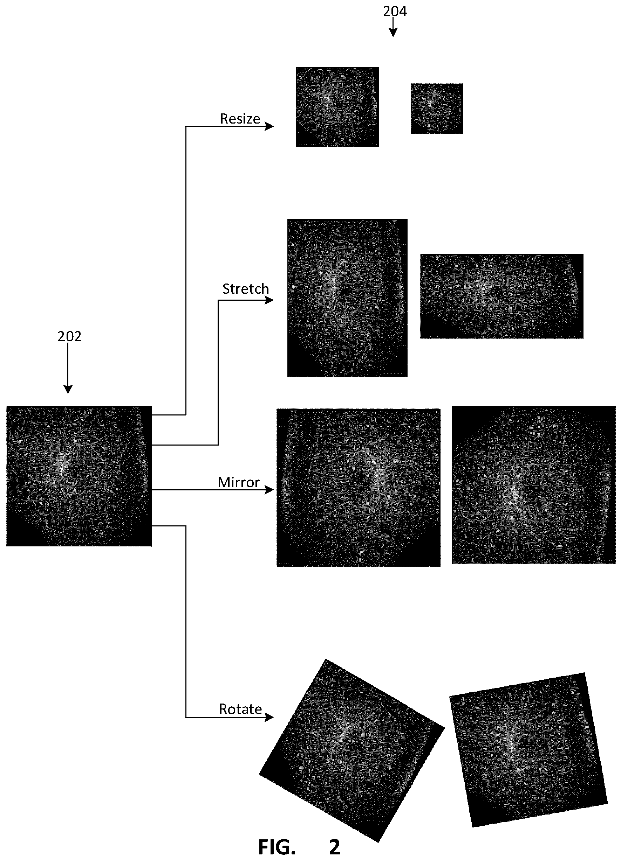

FIG. 2 depicts generating additional training images. In order to ensure a model is not over-fit to available data, original training images may be processed in order to generate additional training images. As depicted in FIG. 2, an initial training image 202 may be used to generate a plurality of additional training images 204. The additional training images 204 are depicted as being generated by resizing the initial image 202, stretching the initial image 202, mirroring the initial image 202 and rotating the initial image 202. Although the transformations depicted in FIG. 2 are depicted as being applied to the initial image individually, the multiple transformations may be applied to the image together, for example, the initial image may be mirrored and stretched, or rotated, resized and stretched, etc.

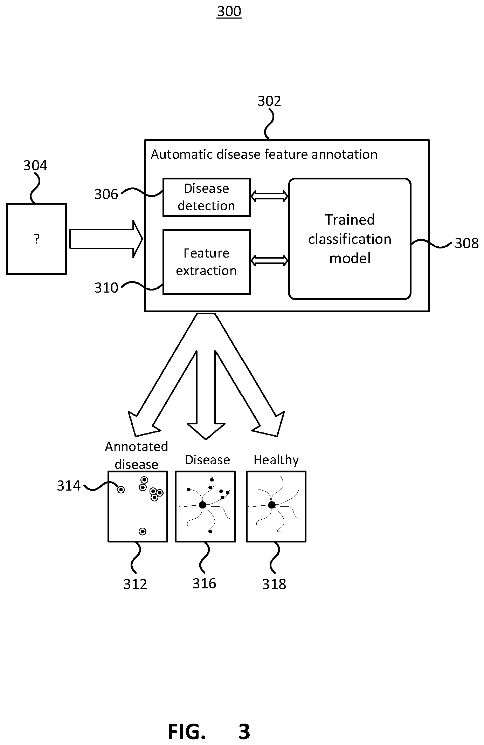

FIG. 3 depicts automatic disease feature annotation functionality. Although not depicted in FIG. 3, the automatic disease feature annotation functionality 302 can be implemented by one or more computer systems comprising one or more processors executing instructions stored in one or more memory units that configure the computer systems to implement the functionality. The automatic disease feature annotation functionality 302 can process one or more input images 304. The input image 304 is a medical image such as an image of the eye, or part of the eye; however, other medical images can be used including for example, ultrasound images, MRI images, x-ray images, light microscopy, 2-photon microscopy, confocal microscopy, optical coherence tomography, photoacoustic imaging, histological slide, etc. The image 304 can be processed by disease detection functionality 306 that determines the presence or absence of a particular disease a trained classification model 308 has been trained to identify. The trained classification model 308 can be trained to classify one or more diseases or conditions. Additionally, although only a single trained classification model is depicted in FIG. 3, it is possible for the disease detection functionality 306 to pass the input image 304 to a plurality of different trained classification models that are trained to detect different diseases/conditions. For example, a first classification model can be trained for identifying features or areas associated with glaucoma, a second classification model can be trained for identifying features or areas associated with diabetic retinopathy, a third classification model can be trained for identifying features or areas associated with floaters, etc. The same image may be provided to each one of the trained classification models in order to determine if the image is associated with any of the trained conditions.

The trained classification model 308 receives the input image and provides a classification output indicative of one or more labels that the model is trained to identify. The classification model can be provided by, or based on, various network architectures including for example, xception, resnext, or mnastnet. In order to successfully identify individual features, the classifier should have a high confidence in the classification prediction. The output from the trained model includes an indication of the prediction confidence level or interval. If the prediction confidence is above a first high threshold, such as 95% or higher, for a particular disease label the image 304 can then be processed by feature extraction functionality 310. The feature extraction functionality can use gradient-based techniques to determine the importance of pixels in the input image in arriving at the classification. The feature extraction functionality generates a feature extraction map indicating the impact of changing particular pixel values has on the classification output. The feature extraction map may be an image with the pixel values at each location of the image indicative of the impact changes at the pixel location have on the output classification. The feature extraction map may be generated based on individual pixel values, or the feature map may be generated based groups or regions of pixels. The feature extraction map can be used to automatically annotate the disease features present in the image. As depicted, the automatic disease feature annotation functionality 302 can categorize the image as having a particular disease or condition present 312 as well as highlighting the extracted features as depicted schematically by circles 314. If the prediction confidence is below the high threshold, but above a low threshold for the disease or condition, the automatic disease feature annotation functionality 302 can identify a disease present in the image, but not with a high enough accuracy in order to automatically extract the disease features. In such cases, the automatic disease annotation functionality 302 classifies the image as having the disease 316 but does not annotate any features. The automatic disease annotation functionality 302 can also classify the image as healthy 318 if the output from the trained classification model indicates that it is a healthy image.

The features highlighted by the automatic feature extraction can be used directly as the annotated disease features. Alternatively, the highlighted features can be further processed in order to generate the annotated disease features. The extracted features can highlight features present in the image that are not in fact part of the disease. For example, in images of the eye, the feature extraction can highlight parts of the eye such as the macula, optic nerve, blood vessels etc. along with disease features such as microaneurysms associated with the disease/condition diabetic retinopathy. The extracted features can be processed to remove the non-disease features to provide the annotated disease features. If the annotated disease features differ from the extracted features, the annotated disease features, or the difference(s) between the extracted features and annotated disease features, can be used in training or updating of the trained classification model.

The trained classification model may be used to classify images as a particular disease image or not. It will be appreciated that a single classification model may be trained to classify images as either being healthy or being a single disease image. Additionally or alternatively, a classification model may be trained to classify an image as being one of a plurality different diseases. The trained classification models may be used to annotate disease features in images and the annotated images may be used directly for various purposes such as in screening or diagnosing a patient with the disease, as well as treating or planning a treatment for the disease. Additionally or alternatively, the annotated features in the images may be used to train other models. For example, a segmentation model may be trained using automatically annotated images provided by the classification model. It will be appreciated that the automatically annotated images generated using the classification model may be used for other purposes.

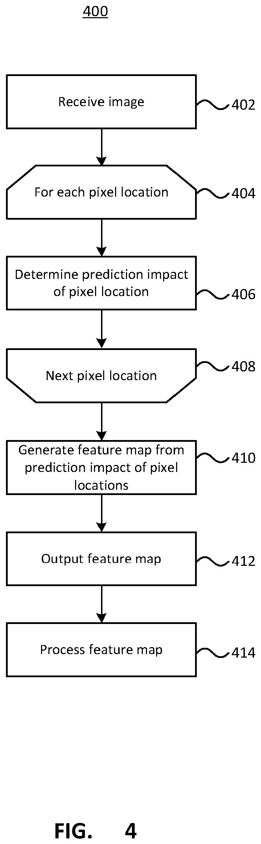

FIG. 4 depicts a method of automatically classifying medical images and extracting features. The method 400 can be performed by a computer system that can receive medical images. The computing system implementing the method 400 can be connected directly to, or be part of the, the imaging system capturing the medical images or can be separate from such imaging systems. Regardless of the particular location or integration of the computing system, the method 400 passes an image to a trained classification model (402). The classification model is trained to classify an image as either being healthy or indicative of a particular disease the model has been trained to recognize. The model can be trained to recognize one or more diseases or conditions. In addition to providing an indication of the disease classification, the model also provides an indication of the confidence that the model's classification is correct. Once the output is received from the classification model it is determined whether the image was classified as healthy or diseased (404). If the image is classified as healthy (Healthy at 404), the method outputs the healthy prediction (406). The model can explicitly classify the image as being healthy. Additionally or alternatively, disease classifications that are below some prediction confidence threshold can be considered as being healthy.

When the image is classified as diseased or not healthy (Diseased at 404), the method 400 determines if the prediction confidence is above a feature extraction threshold (408). In order to properly extract features, it is necessary that the classification of the input image be above a certain confidence level, which can be for example 90%, 95% or higher. The confidence level in the classification prediction necessary in order to extract features can be referred to as an extraction threshold. If the prediction confidence is below the extraction threshold (No at 408) the disease prediction from the classification model is output (410). If however, the prediction confidence is above the extraction threshold (Yes at 408), the method proceeds to extract the features from the image (412). The feature extraction relies upon the classification model in order to identify the features, or portions of the image, the result in the classification and as such in order to provide acceptable feature extraction results, the classification provided by the model must be sufficiently accurate, i.e. have high confidence in the prediction. The extracted features can be provided as a single 2D map or as a plurality of 2D maps. For example, respective 2D feature maps can be generated for red, green, blue (RGB) channels of an image, or other channels depending upon the channels used in the input image. Further, one or more individual 2D maps can be combined together into a 2D map.

Once the features are extracted, the features can be further processed, for example to further identify or annotate the extracted features (414). Where the extracted features can be provided as a 2D map or mask providing locations within the input image that result in the disease classification, annotating the extracted features can result in individual objects each representing a particular feature or group of features. For example, for diabetic retinopathy, an individual annotated feature can be the location within the input image of a micro-aneurism.

The automatically annotated features of one or more of the processed images can be reviewed, for example by a medical professional, and if any features have been misidentified, including for example identifying features that should not have been identified, missing features that should have been identified and/or misidentifying the region of a feature, the feature map can be manually corrected. The corrected feature map can be used for various purposes including for example in the retraining of the model.

Classically training models require additional images of more and more data to train a model if it makes a mistake. Alternatively, the model may continue to be blindly trained or the model structure may be adjusted in an attempt to improve the results. However, by combining a small number of manually annotated images or features, it is possible to effectively direct the attention of the model to evaluate or train on the areas that were specifically missed. The hybrid model training can provide an accurate model capable of automatically annotating features within images with lower training times and lower effort since manually annotating individual features of all training images is not required.

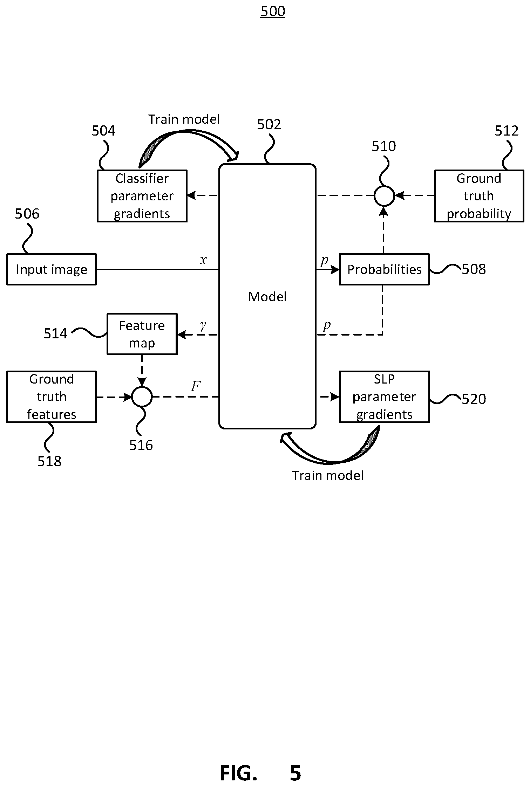

FIG. 5 depicts a process for the hybrid training of the classification model used for feature extraction. The process uses both labelled images as well as corrected feature maps to train the classification model. A limitation of the training approach of using only labelled images is that it is not possible to "spot correct" the results. If a feature is misidentified, for example marks a region where it shouldn't, or misses a region where it should mark, there isn't a way to directly train the model not to make the same mistake. There is only the option to increase the amount of training data, or change the model structure and retrain, then hope it doesn't make the same mistake.

Saliency loss propagation (SLP) is an approach where it is possible to directly train the calculated feature map. Assuming that y has been calculated for every pixel, let *γ** be the ground truth feature/saliency map which is similar to *γ* but corrects for some mistake on some number of pixels. It is possible to then calculate a feature map loss $F = {\sum }_{ij} \left|{γ}_{ij}-{γ}_{ij}^{∗}\right|$ which is a scalar value quantifying the difference between the calculated feature map and the ground truth feature map.

When calculating $F = {\sum }_{ij} \left|{γ}_{ij}-{γ}_{ij}^{∗}\right|$, γ might not necessarily be exactly $\frac{\partial {p}_{k}}{\partial {x}_{ij}}$ but potentially some variation of this which is more useful, such as the integrated gradient as described above. As long as the mathematical operations to get γ are differentiable, then the hybrid training approach can be used. The model parameters *ω*, representing weights of biases of the model, can then be trained on this loss by calculating their respective gradients: $Δ ω = \frac{\partial F}{\partial ω}$

This calculation involves calculating the partial derivative of *F*, which is itself composed of partial derivatives ${γ}_{ij} = \frac{\partial {p}_{k}}{\partial {x}_{ij}}$ making the calculation fairly complex.

As depicted in FIG. 5, a classification model 502 can be trained using classifier parameter gradients 504. The model 502 is provided with an input image 506 x and generates one or more probabilities *p* 508 for the image, with each probability being for whether the image belongs to a particular class. During training, the calculated probability can be combined 510, or compared, to the ground truth probability 512 for the image. The classifier parameter gradients can be determined to minimize the differences between the calculated probabilities and the ground truth probabilities and the classifier parameter gradients used to train the model, for example by adjusting model weightings.

In addition to using the calculated probabilities for training of the model, the model probabilities can be used in calculating the image gradient y for all of the pixels which provide a feature map 514 for the image. One or more of the feature maps generated from the model can misidentify certain features. The generated feature map, which can be incorrect, that is it can incorrectly identify one or more features, can be combined 516 with, or compared to, the ground truth of features for the particular image 518. The incorrect feature map and the corrected ground truth feature map can be used to calculate SLP parameter gradients as a feature map loss gradient using a mean-square loss, $F \left({γ}_{ij}, {γ}_{ij}^{∗}\right) = {\sum }_{ij} {\left|{γ}_{ij}-{γ}_{ij}^{∗}\right|}^{2}$. It will be appreciated that the SLP can use other loss functions such as cross-entropy. More generally, the loss function can be any function such that $F \left({γ}_{ij}, {γ}_{ij}^{∗}\right) = 0$ when ${γ}_{ij} = {γ}_{ij}^{∗}$*,* and increases in magnitude the more different *γᵢⱼ,* ${γ}_{ij}^{∗}$ are. The SLP parameter gradients 520 can then be used to train the model parameters. As described above, the training of the model can be done by calculating the model weights as a partial derivative of the feature map loss gradient *F.*

The hybrid training approach described above allows the classification model to be trained on a number of labelled images, which may be relatively large such as hundreds, thousands, tens of thousands or more. For example, the model can be trained on images that have been identified and labelled as either being 'healthy' or 'diseased' or labelled with particular diseases. Additionally, the model can be trained on a small number of corrected features maps that correct misidentified feature regions.

As described, the hybrid training process can train the classifier model only on non-annotated data and calculates feature maps from the trained classifier model. The training data does not have individual features annotated, but does include a classification label. From the feature maps, a small number of problematic feature maps that have errors in feature detection are identified, and ground truth feature maps are generated that correct for the errors. The model training can continue with the large set of non-annotated data and in parallel with training based on a small number of ground truth feature maps using SLP. The trained model can then be deployed and used. After deployment of the model, users can identify mistakes in the automatically annotated features, and the identified mistakes can then be added to the SLP dataset for case-by-case correction and retraining of the model. A benefit of this approach is it allows for a baseline model to be mainly trained on easily obtained non-annotated data while using only a small number of manually annotated images to correct for individual errors.

FIG. 6 depicts a process for retaining the model. As depicted, an input image 602 can be provided as input to a trained image classifier 604. The classifier can be used to not only classify the input image, but also generate a feature map 606 of one or more features in the image that led to the particular classification. As depicted, the feature map can misidentify one or more features, depicted by arrow 608 as a missing feature. The incorrect feature map can be manually corrected to generate a ground truth feature map 610 that corrects the automatically generated feature map. As depicted by arrow 612, this can include highlighting regions that were not identified, or possibly removing regions that were identified. Regardless, the corrected feature map 610 can be used to retrain the model by calculating the weightings based on the feature map loss between the incorrect feature map 606 and the ground truth feature map 610.

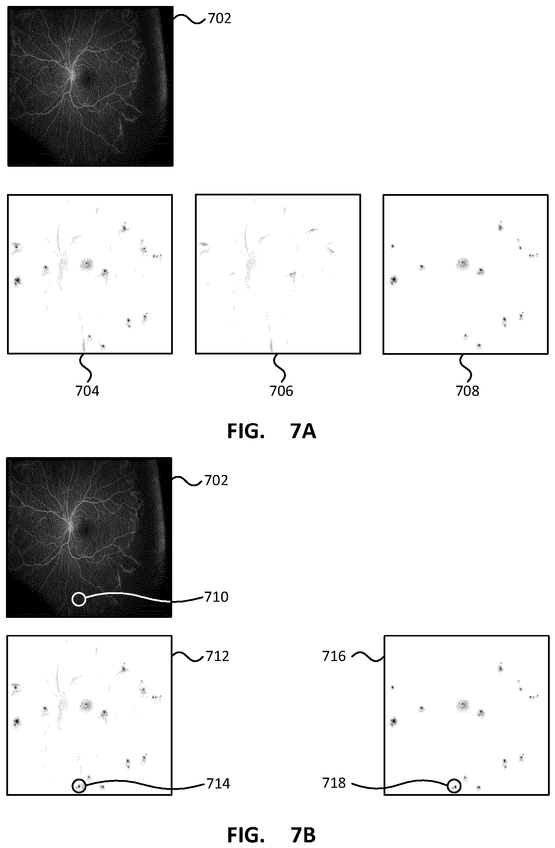

FIG. 7A depicts an image and associated feature map. The image 702 is depicted as an image of a patient with diabetic retinopathy. The image 702 can be processed by the trained classification model in order to generate an initial feature map 704. As depicted the initial feature map 704 may be include both features associated with the disease and non-disease features. The non-disease features may be identified, either from the initial image 702 or the initial feature map 704 using various techniques including using one or more models trained to identify the non-disease features. The non-disease feature 706 may be removed from the initial feature map 704 in order to generate a disease feature map 708. The initial feature map, non-disease feature map, and/or disease feature map may be stored for future use, either in screening, diagnosing, treating or otherwise evaluating the patient, for training and/or retraining one or more models, or for other purposes.

As described above, the classification models used to generate the feature map 704 may incorrectly identify one or more features. As depicted in FIG. 7B, manual annotation may be used to correct the missing features. A professional may evaluate the image 702 in order to identify a region or area that includes a misidentified feature. In addition to identifying the region, the professional can also provide an indication of whether the misidentified feature was not identified in the image, identified as a feature but is not, or too much or too little of the feature area was identified. As depicted a user may indicate an area in the initial image in which a feature was missed, depicted as circle 710. The missing feature may be identified in various ways, possibly by circling or highlighting the area in the image 702. A new feature map 712 of the area 714, and possibly disease feature 716 with the area 718 can be generated. Alternatively, the area in which the missing feature should be located may be specified directly on the feature map 712 or disease map 716. The updated feature map 712 or feature map 717 can then be used as feedback ground truth maps for use in retraining the classification model.

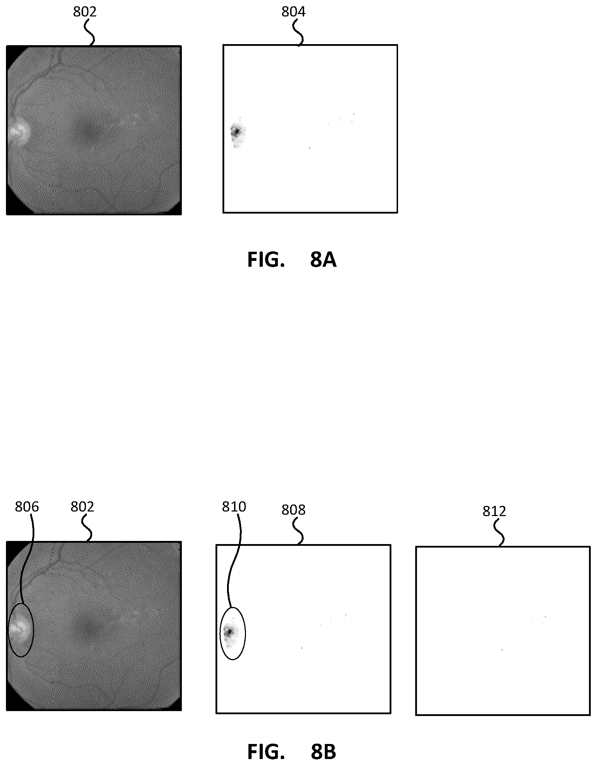

FIG. 8A depicts an image and feature map of a patient with glaucoma. The initial image 802 can be processed in order to generate a feature map 804 of the disease features. As depicted in FIG. 8B, the trained classification or annotation model can identify features or areas that are not associated with the disease. A professional may manually indicate areas that do not include features of the disease. For example, the professional may indicate an area, or areas, of the image 702 depicted by ellipse 806, or possibly on the feature map 808 depicted by ellipse 810 which should not include any disease features. An updated feature map 812 with any features from the indicated areas removed can be generated and used to retrain the classification model as described above.

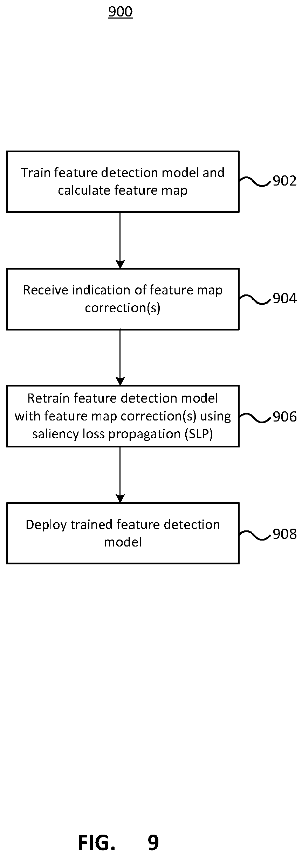

FIG. 9 depicts a method of training a model for automatically annotating images. The method 900 trains a classification model used for feature detection in images. The model can be used to automatically annotate features within images. The model is trained using labelled training images. The training images can be labelled using one of the classes the classification model is being trained to classify. The training of the classification model can be initially trained using typical training techniques for classification models. As part of the training process, the feature detection model is trained by applying the model to the training images to provide a classification result as well as a feature map (902). The classification result is used to train the feature detection model. When training, one or more of the resultant feature maps can be incorrect, for example, certain regions can be identified as a feature that is not a feature or certain features may not have been identified. An indication of feature map correction(s) is received (904). The indication of feature map correction(s) provide an indication of how to correct the incorrect feature map, which can for example comprise an indication of the features that were incorrectly identified. Additionally or alternatively, the indication of the feature map corrections can comprise one or more masks identifying regions where features should not be found in the images. The masks can be generated manually or automatically. For example, other image processing techniques can be used to identify other features which should not be identified by the feature detection model. For example, a feature detection model used to identify treatment regions in a patient's eye can use a mask identifying the patient's retina and veins to remove any features from these regions. The indication of the feature map correction(s) is used to retrain the feature detection model using saliency loss propagation (SLP) (906). The indication of the feature map corrections can include both the use of masks to identify regions that should not include any features, as well as marking of other misidentified features.

The SLP training of the feature detection model can use different functions such as mean-square loss, or cross-entropy loss, etc. For example, assuming *γᵢⱼ* is the i,j pixels of the feature map and ${γ}_{ij}^{∗}$ is the same location for the ground truth feature map, the mean-square loss function $F = {\sum }_{ij} \left|{γ}_{ij}-{γ}_{ij}^{∗}\right|$ subtracts each corresponding pixel and then sums the differences to obtain a total loss, which can then be used in retraining the model. Another approach is if instead of directly defining a ground truth feature map, a "mask" of areas in the image where there shouldn't be detected features can be provided. It is then possible to calculate an exclusion sum: ${F}_{excl} = \sum {γ}_{ij} \left({γ}_{ij}^{∗}=0\right)$ where ${γ}_{ij} \left({γ}_{ij}^{∗}=0\right)$ are the pixels of the feature map where the mask is zero. *F_{excl}* counts the number of pixels outside of the ground truth feature mask. Similarly, it is possible to calculate an inclusion sum: ${F}_{incl} = \sum {γ}_{ij} \left({γ}_{ij}^{∗}=1\right)$, where ${γ}_{ij} \left({γ}_{ij}^{∗}=1\right)$ are the pixels of the feature map where the mask is one, which counts the generated feature map pixels which have not been masked out. **It** is then possible to construct a loss function based on these metrics, such as: $F = \frac{{F}_{excl}}{{F}_{excl} + {F}_{incl}}$ where *F* = 0 if the feature map is entirely within the mask.

The loss function generated from the indication of the feature map correction is used to retrain the feature detection model, which can then be deployed (908). Once deployed, the feature detection model can be applied to images in order to identify features. The identified features can be used for various purposes including for example, screening for a disease, diagnosing disease conditions, identifying treatment locations in a patient's eye, planning a treatment of the patient, and possibly treating the patient. As part of the diagnosis, planning and/or treatment process, the generated feature map of treatment locations can be viewed by a professional and any features can be adjusted. The adjusted features can then be used as feedback for retraining the feature detection model.

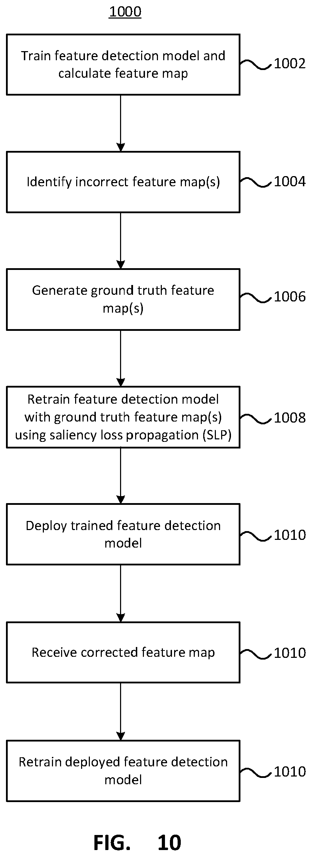

FIG. 10 depicts a further method of training a model for automatically annotating images. The method 1000 trains a classification model used for feature detection. The model is trained using labelled training images and generates feature maps for the training images (1002). One or more incorrect feature maps are identified (1004) and a ground truth, or corrected feature map, is manually generated (1006). The differences between the incorrect feature map and the corrected ground truth feature map can be used to further train the classifier using saliency loss propagation (SLP) (1008). Once trained, the model can be deployed for use (1010). The deployed model can be used in numerous different applications that make use of the feature maps. For example, the feature maps generated from images of eyes with a particular disease can be used in diagnosing a disease, planning treatment of the disease such as laser treatment of the regions identified in the feature map as well as possibly treating the disease.

During use of the model, a user can identify that an automatically generated feature map is incorrect, and can generate a correct feature map. The corrected feature map can be received (1012) and used to retrain the classification model using saliency loss propagation.

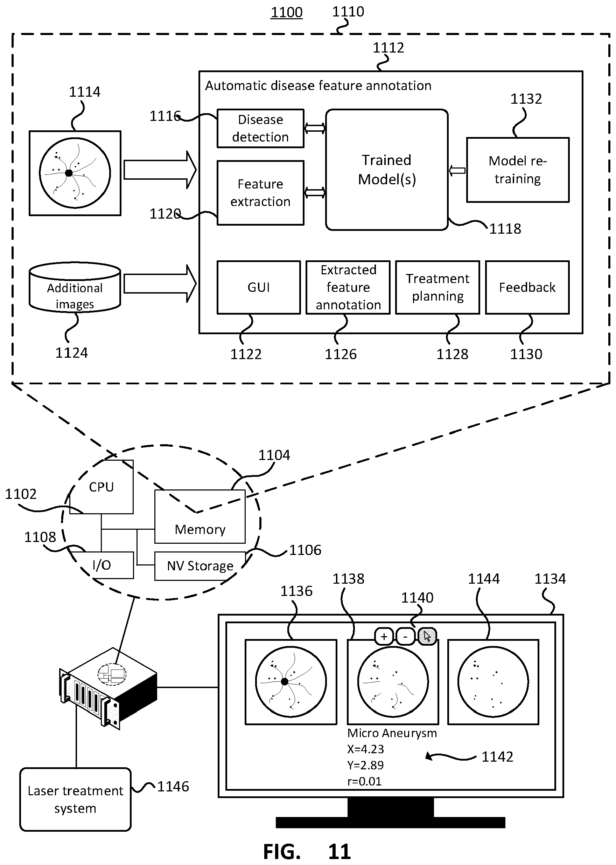

FIG. 11 depicts a system for automatically annotating disease features, planning a treatment of the disease and carrying out the treatment plan. The system 1100 is depicted as comprising a server that implements various functionality. Although depicted as a single server, the functionality or portions of the functionality maybe implemented by a plurality of servers or computing systems The server comprises a CPU 1102 for executing instructions, a memory 1104 for storing instructions, a nonvolatile (NV) storage element 1106 and an input/output (IO) interface for connecting input and/or output devices such as a graphics processing unit (GPU) to the server. The instructions and data stored in the memory 1104, when executed by the CPU 1102, and possibly the GPU configure the server to provide various functionality 1110. Although described as a server, the system 1100 can be implemented by other computing devices, including for example as part of a treatment system such as a laser treatment system 1146, or as dedicated hardware provided by one or more field programmable gate arrays (FPGAs), application specific integrated circuits (ASICs), microprocessors, controllers etc.

The functionality 1110 implemented by the system includes automatic disease feature annotation functionality 1112. The annotation functionality 1112 can receive medical images 1114, depicted as a fundus image of an eye although the functionality can be applied to other types of medical images. Disease detection functionality 1116 can receive the image and pass it to one or more trained classification models 1118 that are trained to classify images as healthy or diseased. The classification models 1118 can be trained on the server or computing device implementing the functionality 1110 or can be trained on one or more separate computing devices and deployed to the server or computing device implementing the functionality 1110, for example possibly using a wired or wireless communication channel. The trained classification model can be further trained with corrected feature maps, either on the server or computing device implementing the functionality 1110 or on another separate computing device.

In addition to providing an indication of the image classification, the trained model 1118 also provides an indication of the prediction confidence of the classification of the trained model 1118. If the prediction confidence is above a feature extraction threshold, which can be for example 95% or higher, feature extraction functionality 1120 can further process the image to extract features. As described above, the feature extraction can use the trained classification model as well as input modification in order to identify the features in the image.

The extracted features, which can be provided as a 2D map highlighting locations within the image that impact the classification results, can be further processed. For example, graphical user interface (GUI) functionality 1122 can process the extracted features to generate a GUI that displays the extracted features, or a representation of the extracted features. The GUI provided by the GUI functionality 1122 can also provide additional functionality, for example it can provide the ability to interact with the features including possibly manually adding, removing, or adjusting the features, as well as displaying other information such as patient details, original images, other medical images 1124, etc. Although depicted as a computer display, the GUI can be presented in other ways, including on a headset, a virtual reality headset, a heads-up display, an augmented reality display or headset etc.

The extracted features can also be processed by extracted feature annotation functionality 1126. While the extracted features highlighted by the feature extraction functionality 1120 provide indications of important features or regions the trained model used to classify the image as diseased, the extracted features can include features that are not disease features but rather common features to the organ being imaged, such as the eye. These common features can be identified using trained models that have been trained to identify the common features, for example using images with and without the common feature present. Further, the extracted features are provided as a 2D image map which highlights the locations of the features in the image, however it does not provide individual features. The extracted feature annotation functionality 1126 can identify individual features from the extracted features and generate corresponding individual annotated features. The extracted feature annotation functionality 1126 can process the extracted feature map to identify the individual features using various techniques including for example image processing techniques that can process the 2D feature map, and possibly the input image, to separate individual features. Once the individual features are identified, corresponding individual annotated features can be generated including information about the annotated feature such as the location within the image, the size and or shape of the annotated feature, an identifier and/or name, notes or comments about the annotated feature, etc. The extracted feature annotation functionality can generate annotated features corresponding to each of the individual extracted features, or can generate annotated features corresponding to a subset of the extracted features such as only those individual features that are not common to imaged organ. That is, common features such as blood vessels, optic nerves, etc. may not be processed to corresponding annotated features. Additionally or alternatively, the extracted feature annotation functionality can include functionality for manually adding/removing annotated features.

The extracted features, or the annotated features generated from the extracted features can be processed by treatment planning functionality 1128. The treatment planning functionality can utilize machine learning techniques to identify portions of the extracted and/or annotated features that can be treated. The treatment planning functionality can utilize additional information, such as additional medical images 1124, in planning the treatment. For example, in treating an ocular condition, a fundus image can be processed in order to identify features that can be treated and additional images can identify additional information such as a thickness of the retina that can help select a subset of the features for actual treatment.

Feedback functionality 1130 can generate feedback that can be used, for example by model re-training functionality 1132, or other models, such as those used in treatment planning or annotating extracted features. The feedback can be generated in various ways. For example, the feedback can be generated directly from manual interactions of a user such as manually removing features or annotated features. The feedback can be generated by comparing a generated treatment plan, which can provide an indication of the important features for treating the condition of disease, to the extracted features of the feature map. The feedback can be used to train or adjust the classification model in order to classify the images based on only those features that can be treated. The re-training can use saliency loss propagation (SLP) as described above. The corrected feature map provided by the feedback can be compared to the automatically generated feature map in order to generate a feature map loss which is a scalar value quantifying the difference between the automatically generated feature map and the corrected feedback feature map. The feature map loss can be used in training the classification model by calculating new weightings based on a gradient of the feature map loss.

As depicted, the system 1100, can include a display or monitor 1134 for displaying a GUI that allows an operator to interact with the system. **It** will be appreciated that the GUI depicted in FIG. 11 is only illustrative and an actual GUI can present desired information in a wide range of formats. As depicted the GUI can display various information including an input image 1136, which is depicted as a fundus image of the eye although other medical images can be used. The GUI can include an image of the individual annotated features 1138. The GUI can provide controls 1140 that allow the operator to interact with the individual annotated features. For example, the controls can allow the operator to select an individual annotated feature and adjust information 1142, such as its location, size, shape, name, notes, etc. Additionally, the controls can include functionality to allow the operator to remove an annotated feature, or possibly add or define new annotated features. The functionality for modifying annotated features can provide functionality to allow an operator to manually add, remove or modify annotated features. Additionally or alternatively, the functionality for modifying annotated features can perform the modifications automatically or semi-automatically for example requiring some user input to define a general region of a possible annotated feature to be modified and/or confirming or rejecting possible modifications. The GUI can also display a treatment plan 1144 for treating the condition. Although not depicted in FIG. 11, the GUI can provide controls to the operator for adjusting the treatment plan. The GUI can provide indications of any of the changes made by the operator to the feedback functionality in order to possibly adjust how features are identified and/or annotated.

The system 1100 can also be coupled to a treatment system 1146, which is depicted as being a laser treatment system, although other treatment systems can be used. The treatment system can carry out the treatment plan for example by treating the determined location with the laser. The treatment system can also include imaging functionality that captures images of the patient that can be processed by the feature annotation functionality. The feature annotation can be implemented by the treatment system 1146. Depending on the computational resources available at the treatment system 1146, or computing device in communication with the treatment system 1146, it is possible for the feature annotation model to process images at a frame rate at which images are captured and as such the model can annotate features in the image frames in real-time during treatment processes. Additionally or alternatively, the treatment system can communicate captured images to a remote computing system that implements the feature annotation functionality, and/or the functionality for training the annotation model. The remote computing system can be in communication with the treatment system using a wired and/or wireless communication channel.

The above has depicted the various functionality being provided by a single server that can be directly connected to a treatment system 1146. The functionality can be provided by one or more networked systems. For example the disease detection functionality 1116, trained models 1118, and feature extraction functionality 1120, the feedback functionality 1130 and the model re-training functionality 1132 can be implemented in one or more cloud servers that can be accessed by different professionals, possibly for a fee. The cloud based functionality can interact with other computer systems or controllers such as controllers of treatment systems. Further still, the results of the feature extraction can be used to identify features to be treated or the output can be provided as input to other systems, for example for training other models, etc.

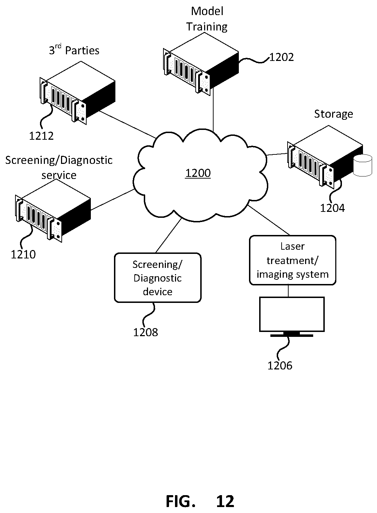

FIG. 12 depicts a system incorporating the hybrid classifier training. The system 1200 comprises a number of computing devices 1202 - 1212 that can be communicatively coupled with one or more of each other. The communication method is depicted as a network 1214 and may be provided by one or more wired and/or wireless communication methods. Although depicted as being connected to communication network 1214 it will be appreciated that the individual computing depicted may communicate with one or more other computing devices directly using wired and/or wireless communication channels.

As depicted, the computing devices may include one or more computing devices providing hybrid model training functionality 1202 as described above. The computing device may receive training data from one or more sources and generates one or more trained classification models that can be deployed to one or more devices. Although depicted as a single computing device 1202, it will be appreciated that multiple different computing devices 1202 may be used to train either the same classification model or different classification models. The trained models, as well as possibly the images used to train and retrain the models may be stored in a data store 1204.

The trained models may be deployed to one or more devices, such as a laser treatment and imaging system 1206 which can be used to both image and treat a patient for the diseases. The treatment and imaging system 1206 can use the trained disease model to screen, diagnose, plan a treatment, and/or treat a patient for the disease or disease the model is trained on. A professional using the system 1206 may adjust one or more locations of feature maps and the information may be provided back to the model training functionality of computing device 1202. Additionally or alternatively, the laser treatment system 1206 may comprise functionality for training and/or retraining the models used. The patient data, and possibly the retraining information may be stored at the laser treatment system or at a data store 1204.

The trained model, or models, may also be provided to a screening or diagnostic device 1206 that may comprise for example a device similar to the laser treatment and imaging system 1206, but without the treatment functionality, or possibly as a low cost device such as a headset that is able to capture images and execute the trained model on the images. Regardless of the type of device, the screening/diagnostic device may store a trained model and execute the model on captured images in order to screen and or diagnose a patient for one or more diseases. Additionally, the screening/diagnosis functionality may be provided as a service by a computing device 1210 that can receive images captured in various ways or using different devices and can execute one or more of the trained models in order to detect possible diseases as well as provide the feature maps.

The trained model may also be deployed to one or more 3^{rd} party services or computing devices 1212 to make use of the trained models in various ways. Additionally or alternatively, the 3^{rd} party services may provide services used by one or more of the computing devices 1202 - 1210. For example, a 3^{rd} party service could provide the model training computing device 1202 with classified images of diseases for use in training the models, or may be used to provide manual annotations or corrections of incorrect feature maps.

The above has described a hybrid approach to training classification models used in automatically generating feature maps. The hybrid approach uses both labelled images to train the classifier as well as corrected feature maps. The feedback used to train and re-train models can be provided in various ways, including for example through a GUI that allows a user to correct a feature map. The hybrid approach to training models for automatic feature extraction has been described with particular reference to its use with identifying disease features, such as microaneurysms associated with diabetic retinopathy, in images of an eyes. However, it will be appreciated that the hybrid training approach can be used to generate feature maps associated with different types of images.

The hybrid training approach can also be used in training models for feature extraction when training data is limited. For example, if the goal is to train a very accurate image classifier, a smaller dataset along with feature masks for these images could provide equivalent accuracy compared to a much larger image dataset alone. This is because the feedback from the ground truth feature mask provides additional constraints, forcing the model to generalize. This can be viewed as training the "attention" or "focus" of the model. It should be noted that attention is a term already used in training AI**,** but is calculated in a very different way. In those circumstances, attention masks are typically trained as intermediate stages or weights in a model. The attention masks or layers are then used to amplify or attenuate signals propagating through the network.

The SLP-based training can be further generalized further by calculating additional gradients. While y is calculated as a gradient of the feature map loss, it is possible to calculate a further gradient of *γ* with respect to the inputs x. This is equivalent to a measurement of where the model is looking in the image in order to decide where to focus its attention, and may not necessarily be the object of interest itself. For example, detecting a volleyball on a beach should show the volleyball itself as the feature map, but $\frac{\partial γ}{\partial x}$ can be more interested in the context of the image, such as a beach in the background or people jumping. Additional gradients can be taken and used in the training and re-training of the classification models.

As described above, a classifier can be trained to classify images. In addition to classifying images, the trained classifier can also be used to identify disease features associated with the particular classification. The approach described above provides a model that can be used to automatically annotate features within images without requiring the time consuming, and possibly difficult, task of manually annotating features for training images. The automatically annotated features can be used for various functionalities, including for example for providing annotated sets of images, identifying disease features within an image of a patient, diagnosing diseases in images, planning a treatment of a disease for a patient, among other reasons.

It will be appreciated by one of ordinary skill in the art that the system and components shown in Figures 1 - 12 can include components not shown in the drawings. For simplicity and clarity of the illustration, elements in the figures are not necessarily to scale, are only schematic and are non-limiting of the elements structures. It will be apparent to persons skilled in the art that a number of variations and modifications can be made without departing from the scope of the invention as defined in the claims.

Although certain components and steps have been described, it is contemplated that individually described components, as well as steps, can be combined together into fewer components or steps or the steps can be performed sequentially, non-sequentially or concurrently. Further, although described above as occurring in a particular order, one of ordinary skill in the art having regard to the current teachings will appreciate that the particular order of certain steps relative to other steps can be changed. Similarly, individual components or steps can be provided by a plurality of components or steps. One of ordinary skill in the art having regard to the current teachings will appreciate that the components and processes described herein can be provided by various combinations of software, firmware and/or hardware, other than the specific implementations described herein as illustrative examples.

The techniques of various embodiments can be implemented using software, hardware and/or a combination of software and hardware. Various embodiments are described above that are directed to systems, methods and devices. It will be appreciated that functionality and features described with particular reference to one embodiment can be combined with features and/or functionality described with reference to another embodiment. Various embodiments are also directed to non-transitory machine, e.g., computer, readable medium, e.g., ROM, RAM, CDs, hard discs, etc., which include machine readable instructions for controlling a machine, e.g., processor, such as a central processing unit (CPU) and/or a graphics processing unit (GPU), to implement one, more or all of the functionality and/or steps of the described method or methods.

Some embodiments are directed to a computer program product comprising a computer-readable medium comprising code for causing a computer, or multiple computers, to implement various functions, steps, acts and/or operations, e.g. one or more or all of the steps described above. Depending on the embodiment, the computer program product can, and sometimes does, include different code for each step to be performed. Thus, the computer program product may, and sometimes does, include code for each individual step of a method, e.g., a method of operating a computing device(s). The code can be in the form of machine, e.g., computer, executable instructions stored on a computer-readable medium such as a RAM (Random Access Memory), ROM (Read Only Memory) or other type of storage device. In addition to being directed to a computer program product, some embodiments are directed to a processor configured to implement one or more of the various functions, steps, acts and/or operations of one or more methods described above. Accordingly, some embodiments are directed to a processor, e.g., CPU and/or GPU, configured to implement some or all of the steps of the method(s) described herein. The processor(s) can be for use in, e.g., a computing device or other device described in the present application.

Numerous additional variations on the methods, systems and apparatus of the various embodiments described above will be apparent to those skilled in the art in view of the above description.

## Claims

1. A method of training a classification model used for feature detection comprising:
training a classifier (102, 502, 604) used for feature detection using a plurality of images (202, 204, 506, 602, 702, 802) and automatically generating respective feature maps (514, 606, 704, 804) of each of the plurality of images using the one or more classifiers (102, 502, 604), wherein the plurality of images (202, 204, 506, 602, 702, 802) does not have individual features annotated, but does include a classification label;
receiving an indication of one or more feature map corrections for one or more of the generated feature maps (514, 606, 704, 804) associated with respective images; and
retraining the classifier model (102, 502, 604) using saliency loss propagation (SLP) with a loss function based on the generated feature map (514, 606, 704, 804) and the indication one or more of the feature map corrections,
generating a correction feature map (610, 708, 812) based on the received indication of one or more feature map corrections,
wherein the loss function quantifies a difference between the automatically generated feature map (514, 606, 704, 804) and the correction feature map (610, 708, 812),
wherein the loss function is $F \left({γ}_{ij}, {γ}_{ij}^{∗}\right)$, and:
$F \left({γ}_{ij}, {γ}_{ij}^{∗}\right) = 0 ,$ when ${γ}_{ij} = {γ}_{ij}^{∗}$; and
$\left|F\left({γ}_{ij}, {γ}_{ij}^{∗}\right)\right|$ increases, as *γᵢⱼ* and ${γ}_{ij}^{∗}$ become more different,
wherein the corrected feature map (610, 708, 812) provides a feature mask indicating locations where no features should be located,
wherein $F = \frac{{F}_{excl}}{{F}_{excl} + {F}_{incl}}$, and:
${F}_{excl} = \sum {γ}_{ij} \left({γ}_{ij}^{∗}=0\right) ,$ where ${γ}_{ij} \left({γ}_{ij}^{∗}=0\right)$ are pixels of the generated feature map (514, 606, 704, 804) where the corrected feature map (610, 708, 812) is zero; and
${F}_{incl} = \sum {γ}_{ij} \left({γ}_{ij}^{∗}=1\right)$, where ${γ}_{ij} \left({γ}_{ij}^{∗}=1\right)$ are pixels of the generated feature map (514, 606, 704, 804) where the corrected feature map (514, 606, 704, 804) is 1.

2. The method of claim 1, wherein the indication of one or more feature map corrections comprises a ground truth feature map for the respective image (202, 204, 506, 602, 702, 802) correcting a misidentified feature in the generated feature map (514, 606, 704, 804).

3. The method of claim 2, wherein receiving the indication of the one or more feature map corrections comprises:
identifying the misidentified features in the generated feature map (514, 606, 704, 804).

4. The method of claim 1, wherein each of the plurality of images (202, 204, 506, 602, 702, 802) are associated with ground truth labels of one or more different classes of the classifier (102, 502, 604).

5. The method of claim 1, wherein the automatically generated feature map (514, 606, 704, 804) identifies one or more regions within the corresponding image (202, 204, 506, 602, 702, 802) which are important to a class prediction by the classifier (102, 502, 604).

6. The method of claim 3, wherein the feature map (514, 606, 704, 804) is generated based on an input image gradient provided by: ${γ}_{ij} = \frac{\partial {p}_{k}}{\partial {x}_{ij}}$ where:
*γᵢⱼ* is the image gradient for an image *x* of pixels *xᵢⱼ*; and
*pₖ* is a model output prediction for the class *k.*

7. The method of claim 3, wherein the feature map (514, 606, 704, 804) is generated based on an input image integrated gradient provided by: ${γ}_{ij} = {\int }_{a = 0}^{1} \frac{\partial {p}_{k}}{\partial \left({x}_{ij}\times a\right)} \frac{\partial \left({x}_{ij}\times a\right)}{\partial a} da$ where:
*γᵢⱼ* is the image gradient for an image *x* of pixels *xᵢⱼ*; and
*pₖ* is a model output prediction for the class *k.*

8. The method of claim 1, wherein:
retraining the classifier comprises determining new weighting parameters of the classifier, and
wherein the weighting parameters are determined based on a gradient of the feature map loss defined by: $Δ ω = \frac{\partial F}{\partial ω} ,$
where:
*ω* is the classifier weightings.

9. The method of claim 1, wherein the feature mask is automatically generated.

10. The method of claim 1, wherein:
the trained classifier (106) is used to annotate regions of a part of a patient's body for treatment,
wherein part of the patient's body for treatment is the eye, and
wherein the method further comprises deploying the trained classifier to identify treatment regions within the patient's eye for laser treatment.

11. The method of claim 10, further comprising:
receiving an indication of one or more annotated regions that misidentify treatment regions; and
retraining the trained classifier (106).

12. A non-transitory computer readable medium storing instructions which when executed by a processor of a computing device configure the computing device to perform a method according to any one of claims 1 to 11.

13. A computing device comprising:
a processor for executing instructions; and
a memory storing instructions which when executed by the processor configure the computing device to perform a method according to any one of claims 1 to 11.

## Patentansprüche

1. Verfahren zum Trainieren eines Klassifizierungsmodells, das zur Merkmalsdetektion verwendet wird, umfassend:
Trainieren eines Klassifikators (102, 502, 604), der zur Merkmalserkennung verwendet wird, unter Verwendung einer Mehrzahl von Bildern (202, 204, 506, 602, 702, 802) und automatisches Erzeugen jeweiliger Merkmalskarten (514, 606, 704, 804) jedes der Mehrzahl von Bildern unter Verwendung des einen oder der mehreren Klassifikatoren (102, 502, 604), wobei die Mehrzahl von Bildern (202, 204, 506, 602, 702, 802) keine annotierten Einzelmerkmale aufweist, aber ein Klassifizierungskennzeichen beinhaltet;
Empfangen einer Angabe einer oder mehrerer Merkmalskartenkorrekturen für eine oder mehrere der erzeugten Merkmalskarten (514, 606, 704, 804), die mit jeweiligen Bildern assoziiert sind; und
erneutes Trainieren des Klassifikatormodells (102, 502, 604) unter Verwendung von Salienzverlustpropagierung (SLP) mit einer Verlustfunktion basierend auf der erzeugten Merkmalskarte (514, 606, 704, 804) und der Angabe einer oder mehrerer der Merkmalskartenkorrekturen,
Erzeugen einer Korrekturmerkmalskarte (610, 708, 812) basierend auf der empfangenen Angabe einer oder mehrerer Merkmalskartenkorrekturen,
wobei die Verlustfunktion eine Differenz zwischen der automatisch erzeugten Merkmalskarte (514, 606, 704, 804) und der Korrekturmerkmalskarte (610, 708, 812) quantifiziert,
wobei die Verlustfunktion $F \left({γ}_{ij}, {γ}_{ij}^{∗}\right)$ ist, und: $F \left({γ}_{ij}, {γ}_{ij}^{∗}\right) = 0 ,$ wenn ${γ}_{ij} = {γ}_{ij}^{∗}$; und
$\left|F \left({γ}_{ij}, {γ}_{ij}^{∗}\right)\right|$ zunimmt, wenn γᵢⱼ und ${γ}_{ij}^{∗}$ sich immer stärker unterscheiden,
wobei die korrigierte Merkmalskarte (610, 708, 812) eine Merkmalsmaske bereitstellt, die Positionen angibt, an denen sich keine Merkmale befinden sollten,
wobei $F = \frac{{F}_{excl}}{{F}_{excl} + {F}_{incl}}$, und:
${F}_{excl} = \sum {γ}_{ij} \left({γ}_{ij}^{∗}=0\right)$, wobei ${γ}_{ij} \left({γ}_{ij}^{∗}=0\right)$ Pixel der erzeugten Merkmalskarte (514, 606, 704, 804) sind, wobei die korrigierte Merkmalskarte (610, 708, 812) null ist; und
${F}_{incl} = \sum {γ}_{ij} \left({γ}_{ij}^{∗}=1\right)$, wobei ${γ}_{ij} \left({γ}_{ij}^{∗}=1\right)$ Pixel der erzeugten Merkmalskarte (514, 606, 704, 804) sind, wobei die korrigierte Merkmalskarte (514, 606, 704, 804) 1 ist.

2. Verfahren nach Anspruch 1, wobei die Angabe einer oder mehrerer Merkmalskartenkorrekturen eine Ground-Truth-Merkmalskarte für das jeweilige Bild (202, 204, 506, 602, 702, 802) umfasst, die ein falsch identifiziertes Merkmal in der erzeugten Merkmalskarte (514, 606, 704, 804) korrigiert.

3. Verfahren nach Anspruch 2, wobei das Empfangen der Angabe der einen oder der mehreren Merkmalskartenkorrekturen Folgendes umfasst:
Identifizieren der falsch identifizierten Merkmale in der erzeugten Merkmalskarte (514, 606, 704, 804).

4. Verfahren nach Anspruch 1, wobei jedes der Mehrzahl von Bildern (202, 204, 506, 602, 702, 802) mit Ground-Truth-Kennzeichnungen einer oder mehrerer verschiedener Klassen des Klassifikators (102, 502, 604) assoziiert ist.

5. Verfahren nach Anspruch 1, wobei die automatisch erzeugte Merkmalskarte (514, 606, 704, 804) eine oder mehrere Regionen innerhalb des entsprechenden Bildes (202, 204, 506, 602, 702, 802) identifiziert, die für eine Klassenvorhersage durch den Klassifikator (102, 502, 604) wichtig sind.

6. Verfahren nach Anspruch 3, wobei die Merkmalskarte (514, 606, 704, 804) basierend auf einem Eingabebildgradienten erzeugt wird, der bereitgestellt wird durch: ${γ}_{ij} = \frac{\partial {p}_{k}}{\partial {x}_{ij}}$ wobei:
γᵢⱼ der Bildgradient für ein Bild x von Pixeln xᵢⱼ ist; und
pₖ ist eine Modellausgabevorhersage für die Klasse k ist.

7. Verfahren nach Anspruch 3, wobei die Merkmalskarte (514, 606, 704, 804) basierend auf einem integrierten Eingabebildgradienten erzeugt wird, der bereitgestellt wird durch: ${γ}_{ij} = {\int }_{a = 0}^{1} \frac{\partial {p}_{k}}{\partial \left({x}_{ij}\times a\right)} \frac{\partial \left({x}_{ij}\times a\right)}{\partial a} da$ wobei:
γᵢⱼ der Bildgradient für ein Bild x von Pixeln xᵢⱼ ist; und
pₖ ist eine Modellausgabevorhersage für die Klasse k ist.

8. Verfahren nach Anspruch 1, wobei:
das erneute Trainieren des Klassifikators Bestimmen neuer Gewichtungsparameter des Klassifikators umfasst, und
wobei die Gewichtungsparameter basierend auf einem Gradienten des Merkmalskartenverlusts bestimmt werden, der definiert ist durch: $Δ ω = \frac{\partial F}{\partial ω} ,$
wobei:
ω die Klassifikatorgewichtungen sind.

9. Verfahren nach Anspruch 1, wobei die Merkmalsmaske automatisch erzeugt wird.

10. Verfahren nach Anspruch 1, wobei:
der trainierte Klassifikator (106) verwendet wird, um Regionen eines Teils des Körpers eines Patienten zur Behandlung zu annotieren,
wobei der Teil des Körpers des Patienten zur Behandlung das Auge ist, und
wobei das Verfahren ferner Bereitstellen des trainierten Klassifikators umfasst, um Behandlungsregionen innerhalb des Auges des Patienten zur Laserbehandlung zu identifizieren.

11. Verfahren nach Anspruch 10, ferner umfassend:
Empfangen einer Angabe einer oder mehrerer annotierter Regionen, die Behandlungsregionen falsch identifizieren; und
erneutes Trainieren des trainierten Klassifikators (106).

12. Nichtflüchtiges computerlesbares Medium, das Anweisungen speichert, die, wenn sie von einem Prozessor einer Computervorrichtung ausgeführt werden, die Computervorrichtung dazu konfigurieren, ein Verfahren nach einem der Ansprüche 1 bis 11 durchzuführen.

13. Computervorrichtung, umfassend:
einen Prozessor zum Ausführen von Anweisungen; und
einen Speicher, der Anweisungen speichert, die, wenn sie von dem Prozessor ausgeführt werden, die Computervorrichtung dazu konfigurieren, ein Verfahren nach einem der Ansprüche 1 bis 11 durchzuführen.

## Revendications

1. Procédé d'entraînement d'un modèle de classification utilisé pour la détection de caractéristiques, comprenant :
l'entraînement d'un classificateur (102, 502, 604) utilisé pour la détection de caractéristiques à l'aide d'une pluralité d'images (202, 204, 506, 602, 702, 802) et la génération automatique de cartes (514, 606, 704, 804) de caractéristiques respectives de chacune de la pluralité d'images à l'aide du ou des classificateurs (102, 502, 604), la pluralité d'images (202, 204, 506, 602, 702, 802) n'ayant pas de caractéristiques individuelles annotées, mais comprenant une étiquette de classification ;
la réception d'une indication d'une ou de plusieurs corrections de cartes de caractéristiques pour une ou plusieurs des cartes (514, 606, 704, 804) de caractéristiques générées associées à des images respectives ; et
le ré-entraînement du modèle classificateur (102, 502, 604) en utilisant une propagation de perte de saillance (SLP) à l'aide d'une fonction de perte sur la base de la carte (514, 606, 704, 804) de caractéristiques générée et de l'indication d'une ou plusieurs des corrections de carte de caractéristiques,
la génération d'une carte (610, 708, 812) de caractéristiques de correction sur la base de l'indication reçue d'une ou de plusieurs corrections de cartes de caractéristiques,
la fonction de perte quantifiant une différence entre la carte (514, 606, 704, 804) de caractéristiques générée automatiquement et la carte (610, 708, 812) de caractéristiques de correction,
la fonction de perte étant $F \left({γ}_{ij}, {γ}_{ij}^{∗}\right)$, et : $F \left({γ}_{ij}, {γ}_{ij}^{∗}\right) = 0 ,$ quand ${γ}_{ij} = {γ}_{ij}^{∗}$; et
$\left|F \left({γ}_{ij}, {γ}_{ij}^{∗}\right)\right|$ augmente, au fur et à mesure que γᵢⱼ et ${γ}_{ij}^{∗}$ deviennent plus différents,
la carte (610, 708, 812) de caractéristiques corrigée fournissant un masque de caractéristiques indiquant des emplacements où aucune caractéristique ne devrait être située, $où F = \frac{{F}_{excl}}{{F}_{excl} + {F}_{incl}} ,$ et :
${F}_{excl} = \sum {γ}_{ij} \left({γ}_{ij}^{∗}=0\right)$, où ${γ}_{ij} \left({γ}_{ij}^{∗}=0\right)$ sont des pixels de la carte (514, 606, 704, 804) de caractéristiques générée où la carte (610, 708, 812) de caractéristiques corrigée est nulle ; et
${F}_{incl} = \sum {γ}_{ij} \left({γ}_{ij}^{∗}=1\right)$, où ${γ}_{ij} \left({γ}_{ij}^{∗}=1\right)$ sont des pixels de la carte (514, 606, 704, 804) de caractéristiques générée où la carte (514, 606, 704, 804) de caractéristiques corrigée vaut 1.

2. Procédé selon la revendication 1, l'indication d'une ou plusieurs corrections de cartes de caractéristiques comprenant une carte de caractéristiques de vérité de terrain pour l'image respective (202, 204, 506, 602, 702, 802), qui corrige une caractéristique incorrectement identifiée dans la carte (514, 606, 704, 804) de caractéristiques générée.

3. Procédé selon la revendication 2, la réception de l'indication de la ou des corrections de carte de caractéristiques comprenant :
l'identification des caractéristiques incorrectement identifiées dans la carte (514, 606, 704, 804) de caractéristiques générée.

4. Procédé selon la revendication 1, chacune de la pluralité d'images (202, 204, 506, 602, 702, 802) étant associée à des étiquettes de vérité de terrain d'une ou plusieurs classes différentes du classificateur (102, 502, 604).

5. Procédé selon la revendication 1, la carte (514, 606, 704, 804) de caractéristiques générée automatiquement identifiant une ou plusieurs régions à l'intérieur de l'image (202, 204, 506, 602, 702, 802) correspondante qui sont importantes pour une prédiction de classe par le classificateur (102, 502, 604).

6. Procédé selon la revendication 3, la carte (514, 606, 704, 804) de caractéristiques étant générée sur la base d'un gradient d'image d'entrée donné par : ${γ}_{ij} = \frac{\partial {p}_{k}}{\partial {x}_{ij}}$ où :
γᵢⱼ est le gradient d'image pour une image x de pixels xᵢⱼ ; et
pₖ est une prédiction de sortie de modèle pour la classe k.

7. Procédé selon la revendication 3, la carte (514, 606, 704, 804) de caractéristiques étant générée sur la base d'un gradient intégré d'image d'entrée donné par : ${γ}_{ij} = {\int }_{a = 0}^{1} \frac{\partial {p}_{k}}{\partial \left({x}_{ij}\times a\right)} \frac{\partial \left({x}_{ij}\times a\right)}{\partial a} da$ où :
γᵢⱼ est le gradient d'image pour une image x de pixels xᵢⱼ ; et
pₖ est une prédiction de sortie de modèle pour la classe k.

8. Procédé selon la revendication 1 :
le ré-entraînement du classificateur comprenant la détermination de nouveaux paramètres de pondération du classificateur, et
les paramètres de pondération étant déterminés sur la base d'un gradient de la perte de carte de caractéristiques défini par : $Δ ω = \frac{\partial F}{\partial ω} ,$
où :
ω est la pondération du classificateur.

9. Procédé selon la revendication 1, le masque de caractéristiques étant généré automatiquement.

10. Procédé selon la revendication 1 :
le classificateur entraîné (106) étant utilisé pour annoter des régions d'une partie du corps d'un patient à des fins de traitement,
une partie du corps du patient à traiter étant l'œil, et le procédé comprenant en outre le déploiement du classificateur entraîné pour identifier des régions de traitement dans l'œil du patient pour un traitement au laser.

11. Procédé selon la revendication 10, comprenant en outre :
la réception d'une indication d'une ou de plusieurs régions annotées qui identifient incorrectement les régions de traitement ; et
le ré-entraînement du classificateur entraîné (106).

12. Support non transitoire lisible par ordinateur conservant des instructions qui, lorsqu'elles sont exécutées par un ou plusieurs processeurs d'un dispositif informatique, configurent le dispositif informatique pour réaliser un procédé selon l'une quelconque des revendications 1 à 11.

13. Dispositif informatique comprenant :
un processeur servant à exécuter des instructions ; et
une mémoire conservant des instructions qui, lorsqu'elles sont exécutées par le processeur, configurent le dispositif informatique pour réaliser un procédé selon l'une quelconque des revendications 1 à 11.
